# EUROPEAN PATENT SPECIFICATION

(11) **EP 0 978 281 B1**
(45) Date of publication and mention of the grant of the patent: **17.07.2002**
(21) Application number: 97919694.6
(22) Date of filing: 25.04.1997
(51) Int. Cl.: A61K 31/445, A61K 9/08, A61P 27/14

(54) **EYEDROPS CONTAINING A LORATIDINE METABOLITE**
AUGENTROPFEN ENTHALTEND EIN LORATIDINMETABOLIT
COLLYRES CONTENANT UNE METABOLITE DE LORATIDINE

(43) Date of publication of application: 09.02.2000
(73) Proprietor: Schering-Plough Kabushiki Kaisha, Osaka-shi, Osaka 541 (JP)
(72) Inventor: FUJIMOTO, Takashi, Kurita-gun, Shiga 520-30 (JP); OKAZAKI, Kimiya, Yokkaichi-shi, Mie 510 (JP); KAMI, Hiroshi, Otsu-shi, Shiga 520-22 (JP); KASE, Koichiro, Otsu-shi, Shiga 520 (JP); YOSHIZAWA, Eiko, Koga-gun, Shiga 520-33 (JP); SHIBAHARA, Takeshi, Kusatsu-shi, Shiga 525 (JP)
(74) Representative: Isenbruck, Günter, Dr.
(86) International application number: JP9701450
(87) International publication number: WO9848803

(56) References cited:
- WO-A-98/34611
- DE-A- 4 442 999
- JP-A- 4 009 339
- JP-A- 4 264 029
- JP-A- 5 004 918
- JP-A- 6 040 910
- JP-A- 9 124 484
- JP-A- 61 289 087
- JP-T- 61 501 205
- US-A- 4 659 716
- US-A- 5 019 591
- J. OCUL. PHARMACOL., 7(4), (1991), GREGG J. BERDY et al., p. 313-24.

## Description

The present invention relates to ophthalmic solutions containing a Loratadine metabolite as an active ingredient.

### BACKGROUND OF THE INVENTION

It is known that Loratadine, ethyl 4-(8-chloro-5,6-dihydro-11H-benzo[5,6]cyclohepta[1,2-b]pyridin-11-ylidene)-1-piperidinecarboxylate, is useful as a non-sedative antihistaminic and acts as an antiallergic in the treatment of long-standing or climatic allergic rhinitis, chronic urticaria, and the like as described in JP 63-55513 B/1988. We discovered that Loratadine or a derivative thereof may be used as an active ingredient of an ophthalmic solution, as disclosed in JP 7-280759/1995 and WO 97/15307.

### DISCLOSURE OF THE INVENTION

It is an object of the present invention to provide an ophthalmic solution containing a Loratadine metabolite as an active ingredient.

The present invention provides for the first time an ophthalmic solution containing the compound of formula I: 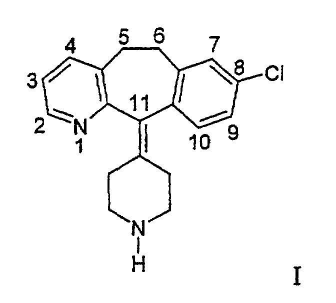 i.e., 8-chloro-6,11-dihydro-11-(4-piperidylidene)-5H-benzo[5,6]cyclohepta[1,2-b]pyridine. Loratadine metabolite is a known compound.

The compound represented by formula I may be easily prepared by heating Loratadine or a derivative thereof in water under an acidic or basic condition. Loratadine and its derivative which are the starting materials of the compound represented by formula I may also be prepared according to, for example, the method as described in JP 63-55513 B/1988.

The ophthalmic solution according to the present invention contains an antihistaminic effective amount of the compound represented by formula I. The antihistaminic effective amount is usually such that a concentration of the compound in the ophthalmic solution is within the range 0.01 to 100 mg/ml.

The ophthalmic solution of the present invention may contain a stabilizer, preservative, isotonic agent, and pH adjusting agent. Examples of the stabilizer include sodium hydrogensulfite, glycerol, sodium edetate, sodium citrate, and butylated hydroxyanisole. Among these sodium edetate is preferred for the reason that it has an excellent inhibitory action on coloration and can maintain a prescribed pH of the solution and the activity of the active compound. As concentration of benzalkonium chloride is increased, the pH of the ophthalmic solution and the activity of the active ingredient are stabilized. Therefore, benzalkonium chloride is preferably incorporated into the ophthalmic solution as a preservative at the maximum concentration described in the positive list of drug additives (0.127%). Examples of the isotonic agent include sodium chloride, D-mannitol, glucose, and glycerol. Among these D-Mannitol is preferred for the reason that it has an excellent inhibitory action on coloration and can maintain a prescribed pH of the solution and the activity of the active compound. These actions are not dependent on the concentration of D-mannitol and thus the ophthalmic solution preferably contains D-mannitol at such a concentration that osmotic pressure of the ophthalmic solution is equal to that of physiological saline, i.e., 1.53%. Examples of the pH adjusting agent include phosphate compounds such as sodium dihydrogenphosphate and disodium hydrogenphosphate, sodium hydroxide, and hydrochloric acid. In order to alleviate irritation on administration, the phosphate concentration is preferably not higher than 0.01M, and it is preferable to use a pH adjusting agent other than phosphate compounds. While the ophthalmic solution may have a pH of about 4 to 8, the pH is preferably brought to about 4 to 5 in order to maintain the active compound to be stable.

The ophthalmic solution of the present invention may contain solubilizers such as Polysorbate® 80, propylene glycol, polyvinyl pyrrolidone K30, and Poloxamer® 188. Among these Polysorbate® 80 is preferred for the reason that it can maintain the ophthalmic solution at an appropriate osmotic pressure.

The ophthalmic solution according to the present invention exhibits an excellent antiallergic activity and are effective on conjunctivitis, pollinosis, vernal conjunctivitis, and the like. In particular, the ophthalmic solution containing the Loratadine metabolite disclosed in the present invention was verified to have an antiallergic activity higher than that containing Loratadine per se as active ingredient. In addition, the ophthalmic solution of the present invention has a high safety since it indicates no disturbance on the cornea, iris or conjunctiva upon instilling in the eye.

The ophthalmic solution according to the present invention is preferably put and stored in a polypropylene container. A container made from polypropylene may keep the ophthalmic solution more stable and thus be more advantageous than those made from any other materials.

The dose of the ophthalmic solution according to the present invention is dependent on the condition being treated. However, it is typically administered 1 to 4 times a day at an amount of 1 to several drops per time.

The present invention will now be illustrated in greater detail by reference to Examples.

### EXAMPLE 1

In about 300 ml of distilled water were dissolved 0.5 g of Loratadine metabolite, 1.0 ml of 10% benzalkonium chloride, 1.27 g of sodium edetate, and 15.3 g of D-mannitol. After addition of 600 ml of distilled water to the solution, 15.6 g of sodium dihydrogenphosphate was added and then the pH of the solution was adjusted to 5.0 using 0.1M disodium hydrogenphosphate. Then, distilled water was added thereto, such that the total volume of the solution is 1000 ml to prepare ophthalmic solution A.

### EXAMPLE 2

Ophthalmic solution B was prepared in the same manner as in EXAMPLE 1 except that 1.0 g of Loratadine metabolite was used.

The results of evaluation of ophthalmic solutions A and B as an ophthalmic preparation are shown in Table 1 below.

**TABLE 1**

| | Solution A | Solution B |
|---|---|---|
| Appearance | glass clear | glass clear |
| APHA Number | 30 | 31 |

### TEST EXAMPLE 1

Loratadine metabolite was evaluated for antihistaminic activity in comparison with Loratadine, as follows.

Rat peritoneal mast cells which were immunized with rat anti-DNP IgE antibody were incubated with the test compound for 15 minutes at 37°C and then stimulated with DNP-Ascaris antigen for 10 minutes after 5 minutes from addition of 10 µg/ml of phosphatidylcholine. Amounts of released or intracellular histamine were measured by means of an autoanalyzer and then % inhibition of histamine release and 50% inhibition concentration (IC₅₀) were determined.

The results thus obtained are shown in Table 2 below. In the table, the value of the % inhibition is expressed in terms of mean (n=5) ± standard error.

**TABLE 2**

| Inhibitory Effect on Histamine Release by Stimulation with Antigen from IgE-immunized Rat Peritoneal Mast Cells | | | |
|---|---|---|---|
| Active Ingredient | Conc. (µM) | % inhibition of Histamine Release | IC₅₀(µM) (95% CL*) |
| | 0.3 | 14.6 ± 6.82 | |
| Loratadine | 1 | 20.9 ± 6.02 | 9.57 |
| Metabolite | 3 | 35.6 ± 7.19 | (3.92-136) |
| | 10 | 49.8 ± 8.40 | |
| | 0.3 | 19.8 ± 5.77 | |
| Loratadine | 1 | 27.1 ± 4.72 | 2.41 |
| | 3 | 45.8 ± 9.26 | (1.14-6.86) |
| | 10 | 73.3 ± 16.8 | |

| | | | |
|---|---|---|---|
| * CL : confidence limit | | | |

## Claims

1. An ophthalmic solution containing a loratadine metabolite represented by formula I

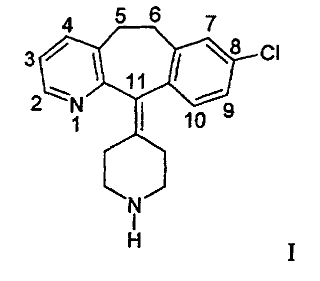

2. The ophthalmic solution according to Claim 1, wherein said ophthalmic solution further contains a stabilizer.

3. The ophthalmic solution according to Claim 2, wherein said stabilizer is sodium hydrogensulfite, glycerol, sodium edetate, sodium citrate, or butylated hydroxyanisole.

4. The ophthalmic solution according to any of the claims 1 to 3, wherein said ophthalmic solution further contains a preservative.

5. The ophthalmic solution according to Claim 4, wherein said preservative is benzalkonium chloride.

6. The ophthalmic solution according to any of the claims 1 to 5, wherein said ophthalmic solution further contains an isotonic agent.

7. The ophthalmic solution according to Claim 6, wherein said isotonic agent is sodium chloride, D-mannitol, glucose or glycerol.

8. The ophthalmic solution according to any of the claims 1 to 7, wherein said ophthalmic solution further contains a phosphate compound at a concentration of not higher than 0.01 M.

9. The ophthalmic solution according to Claim 8, wherein the ophthalmic solution has a pH of 4 to 5.

10. The ophthalmic solution according to any of the claims 1 to 9 wherein said ophthalmic solution is in a polypropylene container.

## Patentansprüche

1. Augentropfen enthaltend einen Loratadin-Metaboliten der Formel I

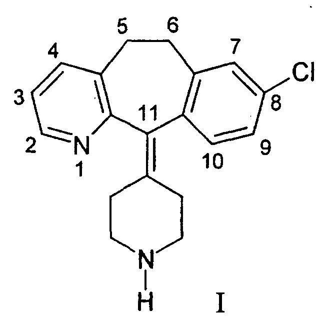

2. Augentropfen nach Anspruch 1, **dadurch gekennzeichnet, dass** die Augentropfen zusätzlich einen Stabilisator enthalten.

3. Augentropfen nach Anspruch 2, **dadurch gekennzeichnet, dass** dieser Stabilisator Natriumhydrogensulfit, Glycerin, Natriumedetat, Natriumcitrat oder butyliertes Hydroxyanisol ist.

4. Augentropfen nach einem der Ansprüche 1 bis 3, **dadurch gekennzeichnet, dass** die Augentropfen zusätzlich ein Konservierungsmittel enthalten.

5. Augentropfen nach Anspruch 4, **dadurch gekennzeichnet, dass** dieses Konservierungsmittel Benzalkoniumchlorid ist.

6. Augentropfen nach einem der Ansprüche 1 bis 5, **dadurch gekennzeichnet, dass** die Augentropfen zusätzlich ein isotonisches Mittel enthalten.

7. Augentropfen nach Anspruch 6, **dadurch gekennzeichnet, dass** dieses isotonische Mittel Natriumchlorid, D-Mannit, Glucose oder Glycerin ist.

8. Augentropfen nach einem der Ansprüche 1 bis 7, **dadurch gekennzeichnet, dass** die Augentropfen zusätzlich eine Phosphatverbindung in einer Konzentration von nicht mehr als 0,01 M enthalten.

9. Augentropfen nach Anspruch 8, **dadurch gekennzeichnet, dass** die Augentropfen einen pH-Wert von 4 bis 5 haben.

10. Augentropfen nach einem der Ansprüche 1 bis 9, **dadurch gekennzeichnet, dass** sich die Augentropfen in einem Polypropylenbehälter befinden.

## Revendications

1. Collyre contenant un métabolite de loratadine représenté par la formule I

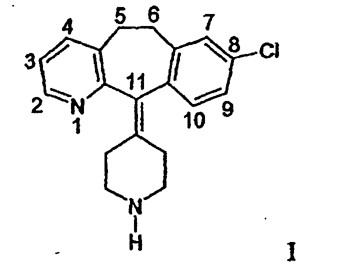

2. Collyre selon la revendication 1, dans lequel ledit collyre contient en outre un stabilisant.

3. Collyre selon la revendication 2, dans lequel ledit stabilisant est l'hydrogénosulfite de sodium, le glycérol, l'édétate de sodium, le citrate de sodium ou l'hydroxyanisole butylé.

4. Collyre selon l'une quelconque des revendications 1 à 3, dans lequel ledit collyre contient en outre un conservateur.

5. Collyre selon la revendication 4, dans lequel ledit conservateur est le chlorure de benzalkonium.

6. Collyre selon l'une quelconque des revendications 1 à 5, dans lequel ledit collyre contient en outre un agent d'isotonicité.

7. Collyre selon la revendication 6, dans lequel ledit agent d'isotonicité est le chlorure de sodium, le D-mannitol, le glucose ou le glycérol.

8. Collyre selon l'une quelconque des revendications 1 à 7, dans lequel ledit collyre contient en outre un composé phosphate à une concentration non supérieure à 0,01 M.

9. Collyre selon la revendication 8, dans lequel le collyre a un pH de 4 à 5.

10. Collyre selon l'une quelconque des revendications 1 à 9, dans lequel ledit collyre se trouve dans un récipient en polypropylène.
